# EUROPÄISCHE PATENTSCHRIFT

(11) **EP 2 922 527 B1**
(45) Veröffentlichungstag und Bekanntmachung des Hinweises auf die Patenterteilung: **12.07.2017**
(21) Anmeldenummer: 13795275.0
(22) Anmeldetag: 26.11.2013
(51) Int. Cl.: A61K 9/14, B07B 7/02, A61K 33/14, A61M 16/10, A61M 11/00, A61K 9/00, A61M 11/02, A61M 15/00, B02C 19/06, B02C 23/30, B02C 23/24

(54) **MIKRONISATOR**
MICRONIZER
MICRONISATEUR

(30) Priorität: 26.11.2012 DE 102012111431
(43) Veröffentlichungstag der Anmeldung: 30.09.2015
(73) Patentinhaber: Klafs GmbH & Co. KG, 74523 Schwaebisch Hall (DE)
(72) Erfinder: BOERMAN, Jan-Kees, 4697 BE Sint-Annaland (NL); ZONNENBERG, Bernardus Cornelis Wilhelmus Maria, 3436 HG Nieuwegein (NL)
(74) Vertreter: Witte, Weller & Partner Patentanwälte mbB
(86) Internationale Anmeldenummer: PCT/EP2013/074731
(87) Internationale Veröffentlichungsnummer: WO 2014/080035

(56) Entgegenhaltungen:
- EP-A1- 1 754 539
- EP-A1- 2 457 559
- EP-A2- 0 139 279
- EP-A2- 1 941 926
- WO-A1-2010/136637
- DE-A1- 19 732 107
- DE-B- 1 270 380
- DE-B- 1 288 890
- GB-A- 468 212
- RO-B1- 122 128
- US-A- 2 561 564
- US-A- 3 734 413
- US-A- 5 747 002

## Beschreibung

Die vorliegende Erfindung betrifft in ihrer allgemeinsten Form das Gebiet der Mühlen, insbesondere Mühlen zur Fein- und Feinstzerkleinerung von anorganischen Salzen. Insbesondere betrifft die vorliegende Erfindung Vorrichtungen und Verfahren zur Herstellung von Partikeln im Mikrometer- sowie Submikrometerbereich, die z.B. zur Herstellung von Aerosolen in medizinischen Anwendungen geeignet sind. Die vorliegende Erfindung betrifft ferner auch Vorrichtungen und Verfahren zur Herstellung von solchen Aerosolen.

Ein Applikationsweg für Medikamente kann die Inhalation eines Aerosols sein, das trockene Partikel mit extrem niedrigen Partikelgrößen des zu verabreichenden Stoffes aufweist. Eine solche Verabreichung kann zum einen bei der Behandlung von Lungenerkrankungen sinnvoll sein, da auf diese Weise ein Wirkstoff direkt am Ort seiner Wirkung appliziert wird, sowie zum anderen dahingehend, dass aufgrund der hohen Durchblutung des Lungengewebes eine sehr schnelle Aufnahme in den Körper erreicht werden kann. In diesem Zusammenhang hat sich z.B. gezeigt, dass durch die Inhalation von äußerst feinen und trockenen Kochsalzpartikeln Linderung bei chronisch pulmonalen Erkrankungen erreicht werden kann.

Um bei der Applikation als Aerosol effektiv zu sein, sollten die im Aerosol vorhandenen trockenen Partikel einen mittleren Teilchendurchmesser von etwa 2µm, vorzugsweise von weniger als 2µm, aufweisen, so dass diese möglichst tief in die Luftwege eindringen.

Zur Erzeugung von Partikeln mit mittleren Teilchendurchmessern im Bereich der oben genannten Werte und insbesondere von Kochsalzpartikeln mit mittleren Teilchendurchmessern im Bereich der oben genannten Werte existieren bereits verschiedene Technologien.

So beschreibt z.B. WO 2005/055984 eine Vorrichtung zum Herstellen eines Salz-Aerosols, bei der der Mahlvorgang durch ein schnell rotierendes Mahlwerkzeug erfolgt.

Die in diesem Dokument beschriebene Vorrichtung hat allerdings den Nachteil, dass hier ein Mahlen mit einer mechanischen Mahlvorrichtung erfolgt, was zum einen im Hinblick auf die Abnutzung der Mahlvorrichtung sowie auf den Eintrag von Verunreinigungen, sei es aus dem Mahlwerkzeug, sei es aus z.B. Schmierstoffen aus dem Antrieb nachteilig ist. Ferner hat sich auch gezeigt, dass solche Vorrichtungen aufgrund der relativ häufigen Wartungsintervalle, die u.a. auch auf die Verarbeitung von korrosivem Kochsalz zurückzuführen sind, für den kontinuierlichen Betrieb nur begrenzt geeignet sind. Abschließend haben diese Vorrichtungen noch den Nachteil, dass diese zu einer hohen Geräuschentwicklung führen, so dass diese z.B. für den Einsatz direkt in der Praxis eines Arztes nicht wirklich geeignet sind.

Die US 5,747,002 beschreibt eine Vorrichtung zur Herstellung eines Salz-Aerosols unter Verwendung eines Strahlmühlenprinzips. Bei dieser Vorrichtung erfolgt das Mahlen im Gegenstromprinzip gefolgt von einer Sichtung basierend auf einem Zyklon.

Auch wenn Strahlmühlen im Allgemeinen einige der oben beschriebenen Nachteile, die mit Mühlen verbunden sind, die auf einem mechanischen Mahlprinzip beruhen, nicht aufweisen, weist die oben beschriebene Vorrichtung zum einen den Nachteil auf, dass diese von ihrer Konstruktion noch immer recht komplex ist und zum anderen, dass dort Gase unter hohem Druck verwendet werden, was zu einem hohen Energieverbrauch führt. Ferner können mit der oben beschriebenen Vorrichtung auch lediglich Partikel mit einem Durchmesser von etwa 7 µm hergestellt werden, welche für den Einsatz in der Medizin nur begrenzt geeignet sind. Abschließend ist auch darauf hinzuweisen, dass in diesem Dokument beschrieben wird, die Partikel nach dem Mahlen direkt einem Vakuumtrocknen unterzogen werden müssen, um ein erneutes Verbacken zu vermeiden, was sowohl die Komplexität der Anlage als auch den Energieverbrauch weiter erhöht.

Die EP 2 457 559 beschreibt eine Vorrichtung zum Mikronisieren eines anorganischen Salzes gemäß dem Oberbegriff des Anspruchs 1. Der zu mikronisierende Feststoff liegt in einer Rückhaltestufe, vor und wird einem Mahlvorgang unterzogen. Eine Trennstufe ist mit der Rückhaltestufe, Mahlstufe und einer Gasversorgung vor der Rückhaltestufe derart angeordnet, dass das Gas die Mahlvorrichtung entlang ihrer Längsachse durchsträmt. Die WO 2008/060173 betrifft ein Verfahren und Vorrichtung zur Bereitstellung eines trockenen Salzaerosols und die therapeutische Anwendung davon. Die Erzeugung des Aerosols beruht auf der Erzeugung von Salzkristallen in einem gesteuerten Kristallisationsvorgang, sowie deren Trocknen, Mahlen und Trennen nach Größe.

Es ist somit eine Aufgabe der vorliegenden Erfindung, verbesserte Vorrichtungen sowie verbesserte Verfahren zur Herstellung von Salzpartikeln zu beschreiben, die z.B. zur Herstellung von Aerosolen verwendet werden können. Insbesondere ist eine Aufgabe der vorliegenden Erfindung, Verfahren und Vorrichtungen zum Herstellen von Salz-Aerosolen für die medizinische Verwendung zu beschreiben.

Die vorliegenden Erfinder haben nun überraschenderweise herausgefunden, dass es durch die Verwendung von sehr trockenen Mahlgasen, z.B. sehr trockener Luft oder sehr trockenen inerten Gasen, möglich ist, Partikel eines anorganischen Salzes, insbesondere Kochsalzpartikel unter Verwendung von Luftströmen mit gegenüber dem bekannten Stand der Technik deutlich reduzierten Drücken und Fließgeschwindigkeiten zu zerkleinern, so dass ein Mahlverfahren entwickelt werden konnte, für die ein deutlich geringerer Energieaufwand notwendig ist. Ferner hat sich dabei auch ergeben, dass die Konstruktion der Mahlvorrichtung gegenüber den bekannten Verfahren deutlich vereinfacht werden kann.

Insbesondere haben die Erfinder herausgefunden, dass es unter Verwendung von sehr trockenen Mahlgasen möglich ist, ein Verfahren bzw. eine Vorrichtung zu konstruieren, in der die zu vermahlenden Partikel durch einen Gasstrom angehoben und verwirbelt werden und dann nach unten fallen, wobei es während des Verwirbelns durch die Interaktion der Partikel untereinander zu einem Mahlvorgang kommt. Das Verfahren bzw. die Vorrichtung haben dabei ferner noch den Vorteil, dass durch den Luftstrom die kleinen und kleinsten Partikel, die während des Mahlvorgangs gebildet werden, aus dem Mahlraum ausgetragen und gleich gesichtet werden können. Es ist klar, dass Verwirbelungen auf verschiedene Weise erzeugt werden können. So ist einerseits der Einsatz von zwei oder mehreren Gasströmen unterschiedlicher Richtung und/oder Stärke denkbar, bevorzugt ist jedoch die Ausnutzung des Coandä-Effekts, bei dem von der Tendenz eines Gases an einer konvexen Oberfläche entlangzulaufen, und somit von der ursprünglichen Strömungsrichtung abgelenkt zu werden, Gebrauch gemacht wird. So kann durch das Einbringen eines oder mehrerer Objekte mit konvexer Oberfläche in einen oder mehrere Gasströme eine Verwirbelung erzielt werden.

Dementsprechend betrifft die vorliegende Erfindung in einem Aspekt eine Vorrichtung gemäß Anspruch 1 zum Mikronisieren eines anorganischen Salzes, die Folgendes aufweist, nämlich eine Mahlvorrichtung, die eine Rückhaltestufe zur Aufnahme des zu mikronisierenden anorganischen Salzes, eine Mahlstufe zur Zerkleinerung des zu mikronisierenden anorganischen Salzes und zur Bildung von mikronisierten Salzpartikeln und eine Trennstufe zur Abtrennung der mikronisierten Salzpartikeln einer gewünschten Größe aufweist, wobei die Rückhaltestufe, die Mahlstufe und die Trennstufe entlang der Längsachse der Mahlvorrichtung linear hintereinander angeordnet sind, sowie eine Gasversorgung zur Bereitstellung eines trockenen Mahlgases, wobei die Gasversorgung so angeordnet und mit der Mahlvorrichtung verbunden ist, dass das trockene Mahlgas entlang der Längsachse der Mahlvorrichtung in die Rückhaltestufe eingeführt wird und die Mahivorrichtung im Wesentlichen entlang der Längsachse die Mahlvorrichtung durchströmt. Die Trennstufe weist einen langgestreckten Hohlkörper auf. In dem Hohlkörper sind Ablenkböden angeordnet, die in alternierender Weise von gegenüberliegenden Seiten in den Hohlkörper hineinragen, wobei die Ablenkböden 50% bis 80% der Querschnittsfläche des Hohlkörpers abdecken, und wobei die Ablenkböden gegenüber einer zur Längsachse der Mahlvorrichtung orthogonalen Ebene unter einem Winkel α von 10° bis 45° stromaufwärts gerichtet geneigt sind.

In einem weiteren Aspekt betrifft die vorliegende Erfindung ein Verfahren gemäß Anspruch 12 zum Mikronisieren eines anorganischen Salzes mit den folgenden Schritten, nämlich
a.) Bereitstellen eines zu mikronisierenden anorganischen Salzes,
b.) Einblasen eines trockenen Mahlgases in das zu mikronisierende anorganische Salz in im Wesentlichen vertikaler Richtung zur Erzeugung von Verwirbelungen in dem zu mikronisierenden anorganischen Salz, wobei ein mikronisierte Salzpartikel aufweisender Gasstrom entsteht, der in einen langgestreckten Hohlkörper eingeführt wird, wobei in dem Hohlkörper Ablenkböden angeordnet sind, die in alternierender Weise von gegenüberliegenden Seiten in den Hohlkörper hineinragen, wobei die Ablenkböden 50% bis 80% der Querschnittsfläche des Hohlkörpers abdecken, und wobei die Ablenkböden gegenüber einer zur Längsachse orthogonalen Ebene unter einem Winkel α von 10° bis 45° stromaufwärts gerichtet geneigt sind,
c.) Austragen der mikronisierten Salzpartikel einer gewünschten Größe aus den in Schritt b.) gebildeten Verwirbelungen in vertikaler Richtung.

Ferner betrifft die vorliegende Erfindung in einem weiteren Aspekt ein NaCl-Aerosol, das mit einer erfindungsgemäßen Vorrichtung oder nach einem erfindungsgemäßen Verfahren hergestellt ist.

Mit der erfindungsgemäßen Vorrichtung bzw. dem erfindungsgemäßen Verfahren wird somit eine Möglichkeit geschaffen, Fein- und Feinstpartikel sowie ein Aerosol von hoher Qualität mit geringem Energieaufwand herzustellen. Die Vorrichtung zeichnet sich ferner noch durch eine hohe mechanische Einfachheit aus, die im extremsten Fall völlig ohne bewegliche Teile auskommen kann, was diese sowohl einfach in der Herstellung wie auch wartungsfreundlich macht und insbesondere auch im Hinblick auf einen kontinuierlichen Betrieb von Vorteil ist.

Unter einem trockenen Mahlgas wird im Sinne der Erfindung jedes Gas verstanden, das nicht mit dem Mahlgut reagiert und das bei 28 °C und 1013 Millibar eine relative Feuchtigkeit von 20 % oder weniger, vorzugsweise von 10 % oder weniger, und besonders bevorzugt von 5 % oder weniger und insbesondere von 1 % oder weniger aufweist. Anders ausgedrückt sind trockene Mahlgase im Sinne der Erfindung Mahlgase, die bei 28 °C und 1013 Millibar einen Wassergehalt von 8000 ppm oder weniger, vorzugsweise 4000 ppm oder weniger, besonders bevorzugt 2000 ppm oder weniger und insbesondere 400 ppm oder weniger aufweisen.

Auch wenn die vorliegenden Erfinder nicht an irgendeine Theorie gebunden sein wollen, gehen sie davon aus, dass die Verwendung von trockenen Mahlgasen mit Feuchtigkeiten in den oben genannten Temperaturbereichen zu einer starken Versprödung innerhalb der Partikel führt, was dann die Zerkleinerung mit Mahlgasen mit relativ wenig kinetischer Energie ermöglicht.

In einer Ausgestaltung der vorliegenden Erfindung weisen die Rückhaltestufe, die Mahlstufe und die Trennstufe keine beweglichen Teile auf.

Diese Ausgestaltung hat zum einen den Vorteil, dass diese aufgrund der mechanischen Einfachheit einfach herzustellen ist, sowie auch, dass diese aufgrund des Fehlens mechanischer Bestandteile und damit verbundener Abnutzung bzw. auch aufgrund der Vermeidung des Eintrags ggf. Eintrag von Hilfsstoffen wie z.B. Schmierstoffen zu einem besonders reinen Gut führen. Ferner sind solche Systeme auch äußerst wartungsfreundlich, was insbesondere für einen Dauerbetrieb vorteilhaft ist.

Bei den Ablenkböden kann es sich um unebene und/oder strukturierte Böden, beispielsweise gewellte oder geriffelte Böden, oder plattenförmige Böden handeln. Falls ein Ablenkboden eine Unebenheit und/oder Strukturierung aufweist, ist diese vorzugsweise derart beschaffen, dass sich dort bei dem Betrieb der Vorrichtung kein Feststoff ansammelt, sondern vielmehr ein Rücklauf in den Gasstrom ermöglicht wird.

Es ist klar, dass ein Ablenkboden nicht den Hohlkörper verschließt, sondern dem Gasstrom einen Durchlass ermöglicht.

In einer weiteren Ausgestaltung sind die Ablenkböden in Richtung der Längsachse der Trennvorrichtung hintereinander angeordnet. Alternativ können mehrere Ablenkböden hintereinander und mehrere Böden alternierend angeordnet sein. Beispielsweise liegen bei einer Gesamtzahl von acht Ablenkböden in Richtung stromabwärts betrachtet zwei Ablenkböden hintereinander, zwei Ablenkböden alternierend, zwei Ablenkböden hintereinander und zwei Ablenkböden alternierend angeordnet vor. Ebenfalls können in Richtung stromabwärts betrachtet vier Ablenkböden hintereinander und zwei Ablenkböden alternierend angeordnet vorliegen. Weitere Kombinationen alternierend und hintereinander angeordneten Ablenkböden, auch mit einer verringerten Anzahl von Ablenkböden, sind denkbar.

Gemäß einer weitern Ausgestaltung können die Ablenkböden Durchgangsöffnungen aufweisen. Die Zahl der Durchgangsöffnungen ist jedoch auf 1-100, 10-90, 20-80, 30-70, 40-60 bzw. 50 begrenzt. Bevorzugt sind jedoch Ablenkböden bei denen keine Durchgangsöffnungen vorliegen.

Weiter bevorzugt sind gewellte, geriffelte oder plattenförmige Böden. Diese weisen vorzugsweise keine Durchgangsöffnungen auf. Besonders bevorzugt ist die Ausgestaltung der Ablenkböden als plattenförmige Böden bzw. Ablenkplatten ohne Öffnungen. Es ist klar, dass die Ablenkböden unterschiedlich ausgestaltet sein können, beispielsweise kann ein Ablenkboden eine gewellte Fläche und ein anderer eine geriffelte Fläche aufweisen.

Alternativ ist eine schraubenförmige bzw. spiralförmige Ausgestaltung der Ablenkböden möglich. Gemäß einer erfindungsgemäßen Alternative liegt ein Ablenkboden vor, worin dieser in dem Hohlkörper derart schraubenförmig bzw. spiralförmig verläuft, dass mindestens eine vollständige Windung um den Innenraum des Hohlkörpers, d.h. 360°, vorliegt. Eine schraubenförmige Ausgestaltung der Ablenkböden kommt vorzugsweise bei einem im Wesentlichen rohrförmigen Hohlkörper mit im Wesentlichen konstanten Querschnitt zum Einsatz. Eine spiralförmige Ausgestaltung der Ablenkböden kann bei einem konischen Hohlkörper verwendet werden.

In einer Ausgestaltung ist die Kante des Ablenkbodens aus einem widerstandsfähigen Material hergestellt, um einen Abrieb durch den mikronisierte Feststoffpartikel aufweisenden Gasstrom zu vermeiden. Geeignete Materialien sind dem Fachmann bekannt und umfassen beispielsweise verschiedene Edelstähle, Bevorzugt ist die Kante des Ablenkbodens stromaufwärts gerichtet abgewinkelt und parallel zur Längsrichtung des Hohlkörpers angeordnet.

In einer weiteren Ausgestaltung liegen die Ablenkböden in voneinander verschiedenen Winkeln α an der Kontaktstelle zu dem Hohlkörper vor. So kann der Ablenkboden, der am weitesten stromaufwärts angeordnet ist, in einem geringen Winkel α bezüglich der orthogonalen Ebene angeordnet sein. Weitere, stromabwärts gerichtete Ablenkböden weisen sukzessiv größere Winkel α auf. Es wurde gefunden, dass hierdurch schneller eine einheitliche Teilchengröße erzielt werden kann. Beispielsweise kann bei einer Abfolge von drei Ablenkböden, der stromaufwärts vorliegende Ablenkboden in einem Winkel α von 15° bis 20° angeordnet sein, der stromabwärts liegende Ablenkboden in einem Winkel α von 30° bis 35° angeordnet sein und der dazwischen liegende Abienkboden in einem Winkel α von 21° bis 29° angeordnet sein. Zur vereinfachten Darstellung werden die Ablenkböden nachstehend mit Großbuchstaben in alphabetischer Folge bezeichnet, wobei der am weitesten stromaufwärts liegende Ablenkboden mit A bezeichnet wird. So können vier Ablenkböden A, B, C, D, fünf Ablenkböden A, B, C, D, E, sechs Ablenkböden A, B, C, D, E, F, sieben Ablenkböden A, B, C, D, E, F,G bzw. acht Ablenkböden A, B, C, D, E, F, G, H vorgesehen sein. Besonders bevorzugt ist dass die einzelnen Ablenkböden A, B, C, D, E, F, G, H individuell die vorstehend beschriebenen Werte für die Winkel an der Kontaktstelle einnehmen können. Der Winkel α für A, B, C, D, E, F, G, H beträgt jeweils 5° bis 45°, bevorzugt sind 5° bis 40°, 5° bis 35°, 5 bis 30°, 6° bis 25°, 7 bis 20°, 8 bis 15°, 9° bis 15°, 10° bis 14°, 11 bis 13°, wobei 1, 2, 3, 4, 5, 6, 7 oder 8 Ablenkböden vorliegen können.

Vorzugsweise sind die Ablenkböden lösbar in dem Hohlkörper befestigt. So können die Ablenkböden individuell an der Wandung des Holkörpers angebracht vorliegen. Bevorzugt ist die Anbringung der Ablenkboden an ein Gestell, dessen Abmessungen den Innabmessungen des Hohlkörpers entsprechen, so dass das Gestell mit den Ablenkböden passend in den Hohlkörper aufgenommen werden kann.

Weiter bevorzugt ist, dass die Ablenkböden mit individuell einstellbarem Winkel α in dem Hohlkörper angeordnet sind. Besonders bevorzugt ist eine Steuerung zur individuellen Einstellung der Winkel α. Von Vorteil ist hierbei, dass in Abhängigkeit der Geschwindigkeit des Gasstroms und der durchschnittlich eingesetzten Teilchengröße verschiedene Winkel α der Ablenkböden eingestellt werden können. So werden bei einer hohen Geschwindigkeit des Gasstroms und einer geringen Teilchengröße, beispielsweise indem ein vorgemahlener Feststoff verwendet wird, vorzugsweise alle Ablenkböden in einem vergleichsweise niedrigen Winkel angeordnet vorliegen, sobald jedoch eine geringe Geschwindigkeit des Gasstroms und eine vergleichsweise hohe Teilchengröße vorliegen, sind die Ablenkböden in einem vergleichsweise großen Winkel α angeordnet. Ferner können die Winkel während dem Verfahren zum Mikronisieren eines Feststoffs geändert werden, um einer verringerten Feststoffgröße Rechnung zu tragen. Mit anderen Worten erlaubt die Steuerung die Anpassung an beispielsweise die durchschnittliche Teilchengröße des eingesetzten Feststoffs.

Alternativ betrifft die vorliegende Erfindung ein Verfahren zum Mikronisieren eines anorganischen Salzes mit den folgenden Schritten, nämlich (a) Bereitstellen eines zu mikronisierenden anorganischen Salzes, (b) Einblasen eines trockenen Mahlgases in das zu mikronisierende anorganische Salz in im Wesentlichen vertikaler Richtung zur Erzeugung von Verwirbelungen in dem zu mikronisierenden anorganischen Salz, wobei mikronisierte Salzpartikel entstehen, (c) Austragen der mikronisierten Salzpartikel einer gewünschten Größe aus den in Schritt (b) gebildeten Verwirbelungen in vertikaler Richtung. Besonders bevorzugt weist das Austragen von Schritt (c) das Einführen eines in Schritt (b) gebildeten und mikronisierte Salzpartikel aufweisenden Gasstroms in einen langgestreckten Hohlkörper auf, wobei in dem Hohlkörper Ablenkböden angeordnet sind, die in alternierender Weise von gegenüberliegenden Seiten in den Hohlkörper hineinragen, wobei die Ablenkböden 50% bis 80% der Querschnittsfläche des Hohlkörpers abdecken, und wobei die Ablenkböden gegenüber einer zur Längsachse orthogonalen Fläche unter einem Winkel α von 10° bis 45° stromaufwärts gerichtet geneigt sind.

Vorzugsweise sind in der Trennstufe bzw. in dem Trennschritt vier bis acht, vorzugsweise vier bis sechs und besonders bevorzugt vier oder fünf Ablenkböden angeordnet. Es ist klar, dass diese Ablenkböden in der vorstehend aufgeführten Weise und mit den entsprechenden Winkeln α angeordnet vorliegen können.

Es hat sich gezeigt, dass eine Trennstufe, die lediglich aus einem langgestreckten Hohlkörper mit darin angeordneten Ablenkböden besteht, zum einen technisch in der Herstellung und in der Wartung extrem einfach ist, jedoch zum anderen zu einer hohen Trennwirkung führen, was insbesondere zu einem vorteilhaften Produkt mit einer sehr engen Korngröi3enverteilung führt.

In einer weiteren Ausgestaltung weist die Gasversorgung einen Kompressor und einen Gastrockner auf.

Es hat sich gezeigt, dass die Verwendung einer Kombination aus einem Kompressor und einem Gastrockner als Gasversorgung insbesondere für den Langzeitdauerbetrieb besonders geeignet ist.

Ein Gastrockner im Sinne der Erfindung kann jede Vorrichtung sein, die bezogen auf eine gegebene Temperatur die relative Feuchtigkeit bzw. den Wassergehalt in einem Gas vermindert. Beispiele für solche Gastrockner sind z.B. Gastrockner, in denen ein Gas heruntergekühlt wird, um die darin enthaltene Feuchtigkeit auszukondensieren, wobei dieses Gas dann in einer trockenen Umgebung wieder z.B. auf Raumtemperatur erwärmen gelassen wird, so dass es nicht zu einer erneuten Aufnahme von Wasser kommt. Ein anderes Beispiel für Gastrockner sind Gastrockner, die auf Absorptionsprinzipien arbeiten, z.B. solche auf Basis von Molekularsieben. Gastrockner auf Basis von Molekularsieben weisen hierbei den Vorteil auf, dass durch periodisches Erhitzen, beispielsweise durch Bestrahlung mit Mikrowellen, die Regeneration der Molekularsiebe erfolgen kann. Bevorzugt werden Zeolithe verwendet. Besonders bevorzugt ist Zeolith A mit einer Porenweite von 3 bis 4 Angström.

In einer weiteren Ausgestaltung der Erfindung weist die Gasversorgung eine Gasflasche mit einem trockenen Gas auf.

Diese Ausgestaltung hat den Vorteil, dass damit auf einfache Weise ein trockenes Gas bereitgestellt werden kann, was insbesondere z.B. bei Betrieb mit geringer Belastung wie beispielsweise in Arztpraxen sinnvoll sein kann.

In einer weiteren Ausgestaltung weist die Vorrichtung ferner eine Zuführvorrichtung zur Zuführung des zu mikronisierenden Feststoffes zu der Rückhaltestufe auf.

Diese Ausgestaltung hat den Vorteil, dass dadurch eine Vorrichtung erhalten werden kann, mit der über lange Zeit im Dauerbetrieb ein gleichmäßig mikronisierter Feststoff bzw. ein Aerosol hergestellt werden kann.

In einer weiteren Ausgestaltung der oben genannten Maßnahme weist die Zuführvorrichtung eine Dosiervorrichtung auf.

Dies hat den Vorteil, dass dadurch der Dauerbetrieb noch besser optimiert werden kann.

In einer weiteren Ausgestaltung ist das trockene Mahlgas Luft, die bei 28 °C und Normaldruck eine relative Luftfeuchtigkeit von höchstens 5 %, vorzugsweise von höchstens 1 %, aufweist. Alternativ ist das trockene Mahlgas ein inertes Gas, das einen Wassergehalt von höchstens 2000 ppm, vorzugsweise von höchstens 400 ppm H₂O aufweist.

Die Erfinder haben herausgefunden, dass unter Verwendung von Mahlgasen mit dieser Trockenheit ein besonders gutes Zerkleinerungsergebnis erzielt werden kann.

In einer weiteren Ausgestaltung des erfindungsgemäßen Verfahrens weisen die in Schritt c.) ausgetragenen mikronisierten Feststoffpartikel eine mittlere Partikelgröße von 3 µm und weniger, vorzugsweise von 2 µm und weniger und insbesondere von 1 µm und weniger auf.

Es hat sich gezeigt, dass Partikel dieser Größe sich insbesondere eignen, Aerosole für medizinische Anwendungen herzustellen.

In einer weiteren Ausgestaltung des erfindungsgemäßen Verfahrens erfolgt dieses kontinuierlich.

Es hat sich gezeigt, dass aufgrund der Einfachheit, insbesondere der mechanischen Einfachheit, der erfindungsgemäßen Vorrichtung das erfindungsgemäße Verfahren ohne Probleme kontinuierlich über einen langen Zeitraum durchgeführt werden kann, wobei zerkleinerte Feststoffpartikel mit gleichbleibend hoher Qualität hergestellt werden können.

In einer weiteren Ausgestaltung ist in dem erfindungsgemäßen Verfahren der zu mikronisierende Feststoff ein anorganisches Salz, das vorzugsweise ausgewählt ist aus der Gruppe bestehend aus NaCl, CaCl₂ und MgCl₂.

Es hat sich gezeigt, dass die oben genannten anorganischen Salze, wobei insbesondere NaCl besonders bevorzugt ist, in mikronisierter Form sich besonders zur Therapie und insbesondere zur palliativen Therapie chronisch pulmonaler Erkrankungen eignen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: schematisch eine Vorrichtung zum Mikronisieren eines Feststoffes,
- Fig. 2: schematisch und im Teilschnitt ein erstes Beispiel einer Mahlvorrichtung für eine Vorrichtung zum Synchronisieren eines Feststoffes, und
- Fig. 3: schematisch und im Teilschnitt eine Explosionsdarstellung eines zweiten Beispiels einer Mahlvorrichtung für eine Vorrichtung zum Mikronisieren eines Feststoffes.

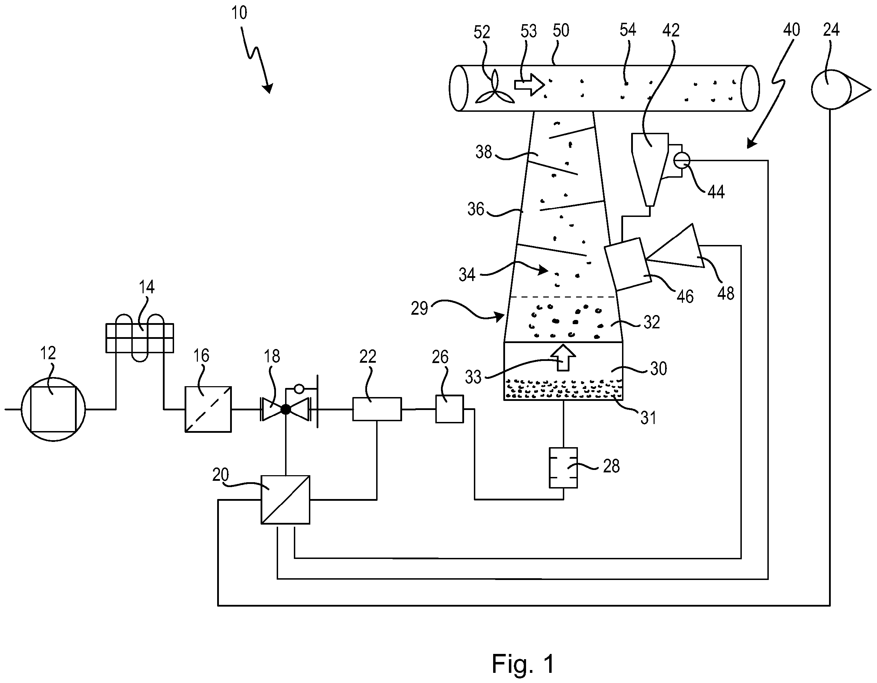

In Fig. 1 ist eine Vorrichtung zum Mikronisieren eines Feststoffes in ihrer Gesamtheit mit dem Bezugszeichen 10 bezeichnet. Die folgende Beschreibung einer Vorrichtung zum Mikronisieren eines Feststoffes erfolgt anschließend entlang des Laufs des Mahlgases.

Die Vorrichtung 10 weist einen Kompressor 12 auf, mit dem ein komprimiertes Mahlgas, im vorliegenden Fall Luft, hergestellt werden kann. Nach dem Durchgang durch den Kompressor wird die Luft einem Kühler 14 zugeführt, der im vorliegenden Fall zur Gastrocknung dient. In diesem Kühler 14 wird die Luft heruntergekühlt, wobei in der Luft vorhandene Feuchtigkeit auskondensiert. Nach Durchgang durch den Kühler 14 wird die Luft in trockener Umgebung wieder erwärmt, so dass keine erneute Feuchtigkeit aufgenommen wird und die Luft gegenüber üblicher Raumluft getrocknet ist. Im vorliegenden Fall weist die Luft, die den Kühler 14 verlässt, bei 28 °C und 1013 Millibar eine Luftfeuchtigkeit von ca. 1 %, was einem Gehalt von etwa 400 ppm H₂O entspricht, auf.

Nach Durchgang durch den Kühler wird die nun getrocknete Luft einem Submikronfilter 16 zugeführt, der ggf. noch in der Luft vorliegende feste bzw. flüssige Verunreinigungen entfernt.

Nach dem Durchgang durch den Filter 16 erreicht die trockene und gesäuberte Luft nun ein Ventil 18, das dazu vorgesehen ist, den Gasfluss zu regulieren. Das Ventil 18 ist hierbei mit einem Regler 20 verbunden, der den Gasfluss steuert. Der Regler 20 ist hierbei ferner mit einem dem Ventil 18 nachgeschalteten Flusssensor 22 sowie einem Konzentrationssensor 24 für das am Ende der Vorrichtung gebildete Salz-Aerosol verbunden und steuert u.a. basierend auf den Daten des Flusssensors 22 und des Konzentrationssensor 24 das Ventil 18. Üblicherweise wird in Systemen, wie hier beispielhaft dargestellt, ein Fluss für das Mahlgas von 0,1 bis 30 l/min verwendet.

Nach Passieren des Ventils 18 sowie des Flusssensors 22 wird die trokkene und gereinigte Luft nun einer Öffnung 26 geführt, über die die Luft entspannt wird, wodurch der Gasfluss eingestellt wird, der dann der Mahlvorrichtung zugeführt werden soll.

Nach Durchgang durch die Öffnung 26 erreicht die gereinigte und getrocknete Luft nun eine Fritte 28, die als Gasanschluss zur für die Mahlvorrichtung 29 dient und die dafür sorgen soll, dass das in der Mahlvorrichtung 29 zu zerkleinernde partikuläre Gut nicht in die Gasleitung dringt.

Die Mahlvorrichtung 29 selbst weist eine Rückhaltestufe 30 auf, in der sich das zu zerkleinernde Gut, im vorliegenden Fall grobkörniges NaCl 31, befindet. In vertikaler linearer Richtung schließt sich an die Rückhaltestufe 30 die Mahlstufe 32 sowie die Trennstufe 34 an.

Im Betrieb tritt nun die getrocknete und gereinigte Luft durch die Fritte 28 in die Rückhaltestufe 30 ein, tritt durch das dort enthaltene NaCl 31 hindurch und reißt dieses wie durch den Pfeil 33 angezeigt in Richtung der Mahlstufe 32 mit. Aufgrund der Auslegung der Mahlvorrichtung 29 kommt es auf der Mahlstufe 32 zu Verwirbelungen, die für den Zerkleinerungseffekt sorgen, wobei die Zerkleinerung per se durch Zusammenstoßen der einzelnen Feststoffteilchen erfolgt. Die Zerkleinerung wird durch das Vorhandensein bzw. das Vorbeiströmen der trockenen Luft unterstützt und teilweise erst ermöglicht, da die trockene Luft dem zu mahlenden NaCl 31 eine größere Menge Feuchtigkeit entzieht, wobei die Erfinder davon ausgehen, dass dieses zur Versprödung führt, die dann das Mahlen vereinfachen.

Nach der Mahlstufe 32 wird das nun zerkleinerte NaCl 31 durch das durch die Mahlvorrichtung 29 durchtretende Mahlgas in die Trennstufe 34 eingetragen, wobei es sich bei der Trennstufe 34 um einen langgestreckten, im Wesentlichen zylindrischen, jedoch konisch zulaufenden Körper handelt, in dem verschiedene Ablenkböden in Form von Ablenkplatten angeordnet sind, von denen eine exemplarisch mit dem Bezugszeichen 38 versehen ist. Die Ablenkplatten, die im Winkel zur Längsachse der Mahlvorrichtung 29 angeordnet sind, dienen dazu, den direkten Fluss der zerkleinerten Partikel zu unterbrechen und somit zu einer Sichtung der Partikel nach Größe beizutragen. Es wird ferner davon ausgegangen, dass zumindest die untere, wenn nicht sogar die unteren zwei Ablenkplatten auch in gewissem Maße zur Mahlwirkung beitragen.

An der Mahlvorrichtung ist ferner ein Vorratsbehälter 42 angeordnet, der mit einem Sensor 44 verbunden ist, der die verbleibende Menge des zu zerkleinernden Gutes in dem Vorratsbehälter 42 misst und dieses zur weiteren Regelung an den Regler 20 weitergibt, der zur kompletten Steuerung der Anlage dient. Der Vorratsbehälter 42 ist ferner mit einer Transportschnecke 46 verbunden, über die das zu zerkleinernde Gut in dosierter Weise der Rückhaltestufe zugeführt werden kann. Die Transportschnecke 46 ist ferner mit einem Motor 48 verbunden, der wiederum mit dem Regler 20 in Verbindung steht. Der Regler 20 kann hierbei u.a. den Motor 48 sowohl basierend auf den Daten, die dieser vom Konzentrationssensor 24 wie auch von dem Sensor 44 erhält, steuern, um zum einen dafür zu sorgen, dass stets eine ausreichende Konzentration an Salz-Aerosol gebildet wird, sowie auch zum anderen, dass ggf. bei Leere des Vorratsbehälters 42 das System z.B. heruntergefahren werden kann.

Nachdem das zerkleinerte NaCl 31 aus der Mahlvorrichtung 29 ausgetragen wird, tritt dieses in ein Querrohr 50 ein, das zum Abtransport dient. Zur Unterstützung des Abtransports des zerkleinerten Gutes, im vorliegenden Fall des zerkleinerten NaCl, ist ferner in dem Querrohr 50 ein Ventilator 52 angeordnet, der Luft in Richtung des Pfeils 53 bewegt, wodurch die zerkleinerten Partikel in Form eines Salz-Aerosols 54 aus dem Querrohr ausgetragen werden.

Zur weiteren Verwendung bzw. Verarbeitung des Salz-Aerosols besteht nun die Möglichkeit, die zerkleinerten NaCl-Partikel z.B. über einen Zyklon aus dem Luftstrom abzuscheiden und diese z.B. in Form von Kapseln zur weiteren Verwendung in Inhalatoren abzupacken. Eine weitere Möglichkeit bestünde aber auch darin, das Salz-Aerosol gleich ohne Weiterverarbeitung entweder in Form einer Maske an einen Patienten zu verabreichen oder dieses in einen größeren Therapieraum z.B. auch zur Therapie von mehreren Menschen gleichzeitig einzuleiten.

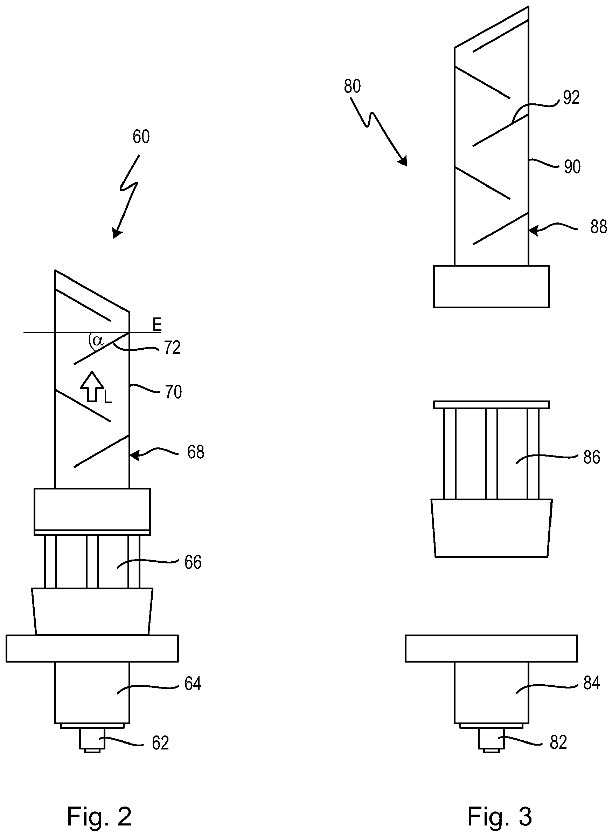

In Fig. 2 ist beispielhaft eine Mahlvorrichtung z.B. zur Verwendung in der in Fig. 1 dargestellten Vorrichtung in ihrer Gesamtheit mit dem Bezugszeichen 60 versehen.

Die Mahlvorrichtung weist wiederum an ihrem unteren Ende einen Gasanschluss 62 auf, der u.a. eine Fritte aufweist, die dafür sorgen soll, dass das zu zerkleinernde Gut nicht in die Gasleitung eintritt. An diesem Gasanschluss 62 schließt sich eine Rückhaltestufe, hier grob gesprochen in Tassenform an, die mit zu mikronisierendem Gut beladen wird.

An die Rückhaltestufe 64 schließt sich in Richtung der Längsachse der Mahlvorrichtung 60 im vorliegenden Fall in vertikaler Richtung eine Mahlstufe 66 an, die hier grob gesprochen eine Käfigform hat. Im Betrieb bilden sich in dieser Mahlstufe Verwirbelungen aus, die dann zur Zerkleinerung des zu mikronisierenden Gutes führen.

An die Mahlstufe 66 schließt sich dann vertikal linear entlang der Längsachse der Mahlvorrichtung eine Trennstufe 68 an, die aus einem im Wesentlichen zylindrischen langgestreckten Hohlkörper 70 besteht, in dem im vorliegenden Fall vier Ablenkplatten angeordnet sind, von denen eine beispielhaft mit dem Bezugszeichen 72 versehen ist. Die Ablenkplatten 72 sind in einem Winkel α von 30° bezüglich der zur Längsachse L der Mahlvorrichtung orthogonalen Ebene E stromaufwärts ausgerichtet.

Im Betrieb funktioniert die Mahlvorrichtung im Wesentlichen wie für die Mahlvorrichtung der Vorrichtung zum Mikronisieren eines Feststoffes wie in Fig. 1 dargestellt. Es ist im vorliegenden Fall vorgesehen, dass diese Mahlvorrichtung diskontinuierlich funktioniert, wobei die Rückhaltestufe 64 einmal mit einem zu zerkleinernden Gut befüllt wird und dann über den Gasanschluss 62 mit einer Quelle für ein trockenes Mahlgas verbunden wird. Das trockene Mahlgas durchströmt in linearer, im vorliegenden Fall in vertikaler Richtung die Rückhaltestufe, wobei dieses das dort gelagerte Mahlgut aufnimmt und in die Mahlstufe 66 einträgt. Durch die Verwirbelungen in der Mahlstufe 66, unterstützt durch die Trockenwirkung des trockenen Mahlgases, wird das Mahlgut dort zerkleinert und in die Trennstufe 68 eingetragen, wobei betont werden muss, dass dieses im vorliegenden Fall immer im Wesentlichen entlang der Längsachse und in vertikaler Richtung geschieht. Allgemein gesprochen und nicht auf die vorliegenden Beispiele begrenzt kann es im Rahmen der vorliegenden Erfindung bevorzugt sein, wenn die Längsachse der Mahlvorrichtung vertikal ist, so dass die Schwerkraft dazu dient, Teile des in die Mahlstufe 66 eingetragenen Mahlgutes wieder in die Rückhaltevorrichtung 64 zurück zu transportieren.

Nach Eintritt in die Trennstufe 68 wird das zerkleinerte Mahlgut dort unter Wirkung der darin angeordneten Ablenkplatten 72 zerkleinert und dann wiederum in vertikaler Richtung ausgetragen, wonach dieses weiterverarbeitet werden kann.

In Fig. 3 ist ein weiteres Beispiel für eine Mahlvorrichtung mit der Ziffer 80 bezeichnet. Die Mahlvorrichtung 80 entspricht im Wesentlichen der in Fig. 2 dargestellten Mahlvorrichtung 60, wobei die Mahlvorrichtung 80 hier in einer Explosionsdarstellung dargestellt ist, die die drei Stufen noch einmal verdeutlichen soll. Hierbei ist wieder eine tassenförmige Rückhaltestufe 84 vorgesehen, die im unteren Ende einen Gasanschluss 82 aufweist. Als Nächstes wiederum linear entlang der Längsachse der Mahlvorrichtung 80 in vertikaler Richtung ist die Mahlstufe 86 dargestellt, gefolgt von der Trennstufe 88, die wiederum aus einem im Wesentlichen zylindrischen langgestreckten Hohlkörper 90 besteht. Der Unterschied zu der aus Fig. 2 bekannten Mahlvorrichtung 60 besteht neben den Abmessungen darin, dass die Trennstufe 88 der Mahlvorrichtung 80 anstatt vier Ablenkplatten fünf Ablenkplatten aufweist, von der wiederum eine mit exemplarisch mit dem Bezugszeichen 92 versehen ist.

Rein beispielhaft weist die Mahlvorrichtung 80 die folgenden Abmessungen auf. Die Tasse der Rückhaltestufe 84 weist eine Höhe von 36 cm und einen Außendurchmesser von 40 cm auf. Die Mahlstufe 86 weist eine Höhe von 85 cm und einen Innendurchmesser von 59,5 cm auf. Die Trennstufe 88 weist eine Gesamthöhe von 146,5 cm auf, wobei der zylindrische Hohlkörper 90 eine Länge von 120 cm und einen Durchmesser von 40 cm aufweist.

## Patentansprüche

1. Vorrichtung zum Mikronisieren eines anorganischen Salzes, die Folgendes aufweist, nämlich eine Mahlvorrichtung (29; 60; 80), die eine Rückhaltestufe (30; 64; 84) zur Aufnahme des zu mikronisierenden anorganischen Salzes, eine Mahlstufe (32; 66; 86) zur Zerkleinerung des zu mikronisierenden anorganischen Salzes und zur Bildung von mikronisierten Salzpartikeln und eine Trennstufe (34; 68; 88) zur Abtrennung der mikronisierten Salzpartikel einer gewünschten Größe aufweist, wobei die Rückhaltestufe (30; 64; 84), die Mahlstufe (32; 66; 86) und die Trennstufe (34; 68; 88) entlang der Längsachse der Mahlvorrichtung (29; 60; 80) linear hintereinander angeordnet sind, sowie eine Gasversorgung zur Bereitstellung eines trockenen Mahlgases, wobei die Gasversorgung so angeordnet und mit der Mahlvorrichtung (29; 60; 80) verbunden ist, dass das trockene Mahlgas entlang der Längsachse der Mahlvorrichtung (29; 60; 80) in die Rückhaltestufe (30; 64; 84) eingeführt wird und die Mahlvorrichtung (29; 60; 80) im Wesentlichen entlang der Längsachse die Mahlvorrichtung (29; 60; 80) durchströmt, wobei die Trennstufe (34; 68; 88) einen langgestreckten Hohlkörper (36; 70; 90) aufweist, wobei in dem Hohlkörper (36; 70; 90) Ablenkböden (38; 72; 92) angeordnet sind,
**dadurch gekennzeichnet, dass** die Ablenkböden (38; 72; 92) in alternierender Weise von gegenüberliegenden Seiten in den Hohlkörper (36; 70; 90) hineinragen, wobei die Ablenkboden (38; 72; 92) 50% bis 80% der Querschnittsfläche des Hohlkörpers (36; 70; 90) abdecken, und wobei die Ablenkböden (38; 72; 92) gegenüber einer zur Längsachse (L) der Mahlvorrichtung orthogonalen Ebene (E) unter einem Winkel α von 10° bis 45° stromaufwärtsgerichtet geneigt sind.

2. Vorrichtung nach Anspruch 1, **dadurch gekennzeichnet, dass** die Rückhaltestufe (30; 64; 84), die Mahlstufe (32; 66; 86) und die Trennstufe (34; 68; 88) keine beweglichen Teile aufweisen.

3. Vorrichtung nach einem der Ansprüche 1 oder 2, **dadurch gekennzeichnet, dass** in der Trennstufe (34; 68; 88) vier bis acht, vorzugsweise vier bis sechs und besonders bevorzugt vier oder fünf Ablenkböden (38; 72; 92) angeordnet sind.

4. Vorrichtung nach einem der Ansprüche 1 bis 3, **dadurch gekennzeichnet, dass** die Ablenkboden (38; 72; 92) uneben, strukturiert oder plattenförmig sind.

5. Vorrichtung nach einem der Ansprüche 1, 3 oder 4, **dadurch gekennzeichnet, dass** die Ablenkböden (38; 72; 92) entfernbar in dem Hohlkörper (36; 70; 90) angeordnet sind.

6. Vorrichtung nach einem der Ansprüche 1 bis 5, **dadurch gekennzeichnet, dass** die Gasversorgung einen Kompressor (12) und einen Gastrockner aufweist.

7. Vorrichtung nach einem der Ansprüche 1 bis 6, **dadurch gekennzeichnet, dass** die Gasversorgung eine Gasflasche mit einem trockenen Gas aufweist.

8. Vorrichtung nach einem der Ansprüche 1 bis 7, **dadurch gekennzeichnet, dass** sie ferner eine Zuführvorrichtung zur Zuführung des zu mikronisierenden anorganischen Salzes zu der Rückhaltestufe aufweist.

9. Vorrichtung nach Anspruch 8, **dadurch gekennzeichnet, dass** die die Zuführvorrichtung eine Dosiervorrichtung aufweist.

10. Vorrichtung nach einem der Ansprüche 1 bis 9, **dadurch gekennzeichnet, dass** das trockene Mahlgas Luft ist, die bei 28 °C und Normaldruck eine relative Luftfeuchtigkeit von höchstens 5 %, vorzugsweise von höchstens 1 % aufweist.

11. Vorrichtung nach einem der Ansprüche 1 bis 9, **dadurch gekennzeichnet, dass** das trockene Mahlgas ein inertes Gas ist, das einen Wassergehalt von höchstens 2000 ppm, vorzugsweise von 400 ppm H₂O aufweist.

12. Verfahren zum Mikronisieren eines anorganischen Salzes mit den folgenden Schritten, nämlich,
a.) Bereitstellen eines zu mikronisierenden anorganischen Salzes,
b.) Einblasen eines trockenen Mahlgases in das zu mikronisierende anorganischen Salz in im Wesentlichen vertikaler Richtung zur Erzeugung von Verwirbelungen in dem zu mikronisierenden anorganischen Salz, wobei ein mikronisierte Salzpartikel aufweisender Gasstrom entsteht, der in einen langgestreckten Hohlkörper eingeführt wird, wobei in dem Hohlkörper (36; 70; 90) Ablenkböden (38; 72; 92) angeordnet sind, die in alternierender Weise von gegenüberliegenden Seiten in den Hohlkörper (36; 70; 90) hineinragen, wobei ein Ablenkboden (38; 72; 92) 50% bis 80% der Querschnittsfläche des Hohlkörpers (36; 70; 90) abdeckt, und wobei die Ablenkböden (38; 72; 92) gegenüber einer zur Längsachse (L) orthogonalen Ebene (E) unter einem Winkel α von 10° bis 45° stromauftnrärtsgerichtet geneigt sind,
c.) Austragen der mikronisierten Salzpartikel einer gewünschten Größe aus den in Schritt b.) gebildeten Verwirbelungen in vertikaler Richtung.

13. Verfahren nach Anspruch 12, wobei das in Schritt b.) eingeblasene, trockene Mahlgas Luft ist, die eine relative Feuchtigkeit bei 28 °C und Normaldruck von höchstens 5 %, vorzugsweise von höchstens 1 % aufweist.

14. Verfahren nach Anspruch 12 oder 13, wobei das in Schritt b.) eingeblasene, trockene Mahlgas ein inertes Gas ist, das einen Wassergehalt von höchstens 2000 ppm, vorzugsweise von 400 ppm H₂O aufweist.

15. Verfahren nach einem der Ansprüche 12 bis 14, **dadurch gekennzeichnet, dass** das Verfahren kontinuierlich erfolgt.

## Claims

1. Device for micronizing an inorganic salt, having the following: specifically a grinding device (29; 60; 80), which has a retaining stage (30; 64; 84) for receiving the inorganic salt to be micronized, a grinding stage (32; 66; 86) for comminuting the inorganic salt to be micronized and for forming micronized salt particles, and a separating stage (34; 68; 88) for separating the micronized salt particles of a desired size, the retaining stage (30; 64; 84), the grinding stage (32; 66: 86), and the separating stage (34; 68; 88) being arranged one behind the other in a linear manner along the longitudinal axis of the grinding device (29; 60; 80), and also a gas supply for providing a dry grinding gas, the gas supply being arranged and connected to the grinding device (29; 60; 80) in such a way that the dry grinding gas is introduced into the retaining stage (30; 64; 84) along the longitudinal axis of the grinding device (29; 60; 80) and flows through the grinding device (29; 60; 80) substantially along the longitudinal axis of the grinding device (29; 60; 80), the separating stage (34; 68; 88) having an elongated hollow body (36; 70; 90), and deflecting plates (38; 72; 92) being arranged in the hollow body (36; 70; 90),
**characterized in that** the deflecting plates (38; 72; 92) project in an alternating manner from opposite sides into the hollow body (36; 70; 90), the deflecting plates (38; 72, 92) covering 50% to 80% of the cross-sectional area of the hollow body (36; 70; 90), and the deflecting plates (38; 72; 92) being angled in an upstream-directed manner at an angle α of 10° to 45° with respect to a plane (E) which is orthogonal to the longitudinal axis (L) of the grinding device.

2. Device according to Claim 1, **characterized in that** the retaining stage (30; 64; 84), the grinding stage (32; 66; 86) and the separating stage (34; 68; 88) have no movable parts.

3. Device according to either of Claims 1 and 2, **characterized in that** four to eight, preferably four to six and particularly preferably four or five, deflecting plates (38; 72, 92) are arranged in the separating stage (34; 68; 88).

4. Device according to one of Claims 1 to 3, **characterized in that** the deflecting plates (38; 72; 92) are uneven, structured or sheet-like.

5. Device according to one of Claims 1, 3 or 4, **characterized in that** the deflecting plates (38; 72; 92) are removably arranged in the hollow body (36; 70; 90).

6. Device according to one of Claims 1 to 5, **characterized in that** the gas supply has a compressor (12) and a gas dryer.

7. Device according to one of Claims 1 to 6, **characterized in that** the gas supply has a gas bottle with a dry gas.

8. Device according to one of Claims 1 to 7, **characterized in that** it also has a feeding device for feeding the inorganic salt to be micronized to the retaining stage.

9. Device according to Claim 8, **characterized in that** the feeding device also has a metering device.

10. Device according to one of Claims 1 to 9, **characterized in that** the dry grinding gas is air which at 28°C and under standard pressure has a relative air humidity of at most 5%, preferably of at most 1%.

11. Device according to one of Claims 1 to 9, **characterized in that** the dry grinding gas is an inert gas which has a water content of at most 2000 ppm, preferably of 400 ppm H₂O.

12. Method for micronizing an inorganic salt with the following steps, specifically
a.) providing an inorganic salt to be micronized,
b.) blowing a dry grinding gas into the inorganic salt to be micronized in a substantially vertical direction to produce vortices in the inorganic salt to be micronized, thereby creating a gas stream having micronized salt particles, which is introduced into an elongated hollow body, deflecting plates (38; 72; 92) which project in an alternating manner from opposite sides into the hollow body (36; 70; 90) being arranged in the hollow body (36; 70; 90), a deflecting plate (38; 72, 92) covering 50% to 80% of the cross-sectional area of the hollow body (36; 70; 90), and the deflecting plates (38; 72; 92) being angled in an upstream-directed manner at an angle α of 10° to 45° with respect to a plane (E) which is orthogonal to the longitudinal axis (L),
c.) discharging the micronized salt particles of a desired size from the vortices formed in step b.) in a vertical direction.

13. Method according to Claim 12, the dry grinding gas that is blown in in step b.) having a relative humidity at 28°C and under standard pressure of at most 5%, preferably of at most 1%.

14. Method according to Claim 12 or 13, the dry grinding gas that is blown in in step b.) being an inert gas which has a water content of at most 2000 ppm, preferably of 400 ppm H₂O.

15. Method according to one of Claims 12 to 14, **characterized in that** the method takes place continuously.

## Revendications

1. Dispositif pour microniser un sel inorganique, qui présente les composants suivants, à savoir un dispositif de broyage (29; 60; 80), qui présente un étage de retenue (30; 64; 84) destiné à contenir le sel inorganique à microniser, un étage de broyage (32; 66; 86) pour le broyage du sel inorganique à microniser et pour la formation de particules de sel micronisées et un étage de séparation (34; 68; 88) pour la séparation des particules de sel micronisées d'une grosseur désirée, dans lequel l'étage de retenue (30; 64; 84), l'étage de broyage (32; 66; 86) et l'étage de séparation (34; 68; 88) sont disposés en ligne l'un derrière l'autre le long de l'axe longitudinal du dispositif de broyage (29; 60; 80), ainsi qu'une source de gaz pour fournir un gaz de broyage sec, dans lequel la source de gaz est disposée et reliée au dispositif de broyage (29; 60; 80) de telle manière que le gaz de broyage sec soit introduit dans l'étage de retenue (30; 64; 84) le long de l'axe longitudinal du dispositif de broyage (29; 60; 80) et traverse le dispositif de broyage (29; 60; 80) essentiellement le long de l'axe longitudinal du dispositif de broyage (29; 60; 80), dans lequel l'étage de séparation (34; 68; 88) présente un corps creux allongé (36; 70; 90), dans lequel des plateaux de déviation (38; 72; 82) sont disposés dans le corps creux (36; 70; 90),
**caractérisé en ce que** les plateaux de déviation (38; 72; 92) pénètrent dans le corps creux (36; 70; 90) de manière alternée à partir de côtés opposés, dans lequel les plateaux de déviation (38; 72; 92) recouvrent de 50 % à 80 % de l'aire de section transversale du corps creux (36; 70; 90), et dans lequel les plateaux de déviation (38; 72; 92) sont inclinés en direction amont de l'écoulement d'un angle α de 10° à 45° par rapport à un plan (E) orthogonal à l'axe longitudinal (L) du dispositif de broyage.

2. Dispositif selon la revendication 1, **caractérisé en ce que** l'étage de retenue (30; 64; 84), l'étage de broyage (32; 66; 86) et l'étage de séparation (34; 68; 88) ne présentent aucune pièce mobile.

3. Dispositif selon une des revendications 1 ou 2, **caractérisé en ce que** quatre à huit, de préférence quatre à six et de préférence encore quatre ou cinq plateaux de déviation (38; 72; 92) sont disposés dans l'étage de séparation (34; 68; 88).

4. Dispositif selon l'une quelconque des revendications 1 à 3, **caractérisé en ce que** les plateaux de déviation (38; 72; 92) sont inégaux, structurés ou en forme de plaque.

5. Dispositif selon l'une quelconque des revendications 1, 3 ou 4, **caractérisé en ce que** les plateaux de déviation (38; 72; 92) sont disposés de façon amovible dans le corps creux (36; 70; 90).

6. Dispositif selon l'une quelconque des revendications 1 à 5, **caractérisé en ce que** la source de gaz présente un compresseur (12) et un sécheur de gaz.

7. Dispositif selon l'une quelconque des revendications 1 à 6, **caractérisé en ce que** la source de gaz présente une bouteille de gaz avec un gaz sec.

8. Dispositif selon l'une quelconque des revendications 1 à 7, **caractérisé en ce qu'**il présente en outre un dispositif d'alimentation pour amener le sel inorganique à microniser à l'étage de retenue.

9. Dispositif selon la revendication 8, **caractérisé en ce que** le dispositif d'alimentation présente un dispositif de dosage.

10. Dispositif selon l'une quelconque des revendications 1 à 9, **caractérisé en ce que** le gaz de broyage sec est de l'air, qui présente à 28°C et à la pression normale une humidité atmosphérique relative de 5 % au maximum, de préférence de 1 % au maximum.

11. Dispositif selon l'une quelconque des revendications 1 à 9, **caractérisé en ce que** le gaz de broyage sec est un gaz inerte, qui présente une teneur en eau de 2000 ppm au maximum, de préférence de 400 ppm H₂O au maximum.

12. Procédé pour microniser un sel inorganique comprenant les étapes suivantes, à savoir:
a) préparer un sel inorganique à microniser,
b) insuffler un gaz de broyage sec dans le sel inorganique à microniser dans une direction essentiellement verticale pour produire des tourbillons dans le sel inorganique à microniser, dans lequel il apparaît un courant de gaz contenant des particules de sel micronisées, qui est introduit dans un corps creux allongé, dans lequel des plateaux de déviation (38; 72; 92) sont disposés dans le corps creux (36; 70; 90), qui pénètrent dans le corps creux (36; 70; 90) de façon alternée à partir de côtés opposés, dans lequel un plateau de déviation (38; 72; 92) recouvre de 50 % à 80 % de l'aire de section transversale du corps creux (36; 70; 90), et dans lequel les plateaux de déviation (38; 72; 92) sont inclinés en direction amont de l'écoulement d'un angle α de 10° à 45° par rapport à un plan (E) orthogonal à l'axe longitudinal (L),
c) extraire les particules de sel micronisées d'une grosseur désirée hors des tourbillons formés à l'étape b) en direction verticale.

13. Procédé selon la revendication 12, dans lequel le gaz de broyage sec insufflé à l'étape b) est de l'air, qui présente une humidité relative à 28°C et à la pression normale de 5 % au maximum, de préférence de 1 % au maximum.

14. Procédé selon la revendication 12 ou 13, dans lequel le gaz de broyage sec insufflé à l'étape b) est un gaz inerte, qui présente une teneur en eau de 2000 ppm au maximum, de préférence de 400 ppm H₂O au maximum.

15. Procédé selon l'une quelconque des revendications 12 à 14, **caractérisé en ce que** le procédé est exécuté en continu.
